# EUROPEAN PATENT APPLICATION

(11) **EP 2 712 656 A2**
(43) Date of publication of application: **02.04.2014**
(21) Application number: 13003617.1
(22) Date of filing: 18.07.2013
(51) Int. Cl.: A61Q 11/00, A61K 8/19

(54) **Composition for preventing the dental calculus formation**

(30) Priority: 18.05.2012 TR 201205824
(71) Applicant: Bortek Bor Teknolojileri Ve Mekatronik Sanayi, Odunpazarl Eskisehir (TR)
(72) Inventor: Nuran, Ay, Eskisehir (TR); Suleyman, Ay, Eskisehir (TR)
(74) Representative: Pipan, Marjan

(57) **Abstract**

The present invention relates to the prevention of dental calculus formation owing to the mixing, with the toothpaste, tooth powder, dental gel and mouthwash, of a composition comprising the nano-sized hexagonal boron nitride, developed for preventing the formation of the dental calculus that accumulates around the teeth and causes the gingival diseases, and the application of the same mixture at regular intervals to the teeth, thereby enabling said nano composition to find its way into the pores on the tooth and into the pores in the tooth plaque.

## Description

The present invention relates to mixing a composition, which is developed for preventing the formation and accumulation of the dental calculus around the teeth and which comprises nano-size hexagonal boron nitride (hBN), with the toothpaste, tooth powder, dental gel and mouthwash, and applying the same mixture at regular intervals to the teeth.

Dental calculus develops in the form of accumulation and deposit in the gum region. Dental calculus forms in two phases. In the first phase, the plaque accumulates and collects on the tooth. The plaque contains inorganic and organic components that are originated from saliva, food and bacteria which present in the oral cavity. In the second phase, the plaque undergoes calcification to form the dental calculus. Initially, the amorphous calcium phosphate deposits begin to develop within the dental matrix. When a sufficient amount of calcium phosphate aggregates have formed, they crystallize to form hydroxyapatite.

The presence of the plaque and the calculus is detrimental to the health of the teeth and the gum. A great variety of chemical and biological materials are recommended to delay the formation of dental calculus and to remove the same once formed. Moreover, the formed dental calculus is mechanically cleaned at certain intervals by the dentists.

In the US Patent No. 4846650, it is stated that polycarboxylic acid and the salts thereof prevent the formation of the dental calculus.

In the US Patent No. 5,028,412, it is disclosed that the aliphatic carboxylic acid forms an aluminum-carboxylate complex with non-poisonous aluminum salts to reduce and control the dental calculus formation.

In the US Patent No. 5,096,701, it is disclosed that a composition comprising sodium tripolyphosphate and ammonium tripolyphosphate provides an effective method for the fight against the dental calculus.

According to the patents mentioned above, the chemical methods are employed for the prevention or resolution of the dental calculus, wherein the calcium ion is gelled and/or the growth of crystals is prevented, thereby impeding the formation of the dental calculus and/or removing the calcium and breaking down the calculus.

With the present invention, the nano-size hexagonal boron nitride (hBN), which is a material completely different than those used in the previous chemical methods, finds its way into the pores on the tooth surface and also into those in the plaque, thereby preventing the formation and crystallization of the dental calculus on the tooth surface. The nano-size hexagonal boron nitride is an inert material, which does not react with and is not wetted by any chemical and which features the superior lubricating ability.

It also protects the teeth surface against the acidic components formed with the saliva. Since it prevents the formation of the dental calculus, it enables to avoid the gingival diseases and tooth decays. Hexagonal boron nitride is available within many cosmetic medical products.

With the present invention, the benefits such as the following may be obtained:
1. Preparing a composition that comprises the nano-size hexagonal boron nitride for the prevention of the dental calculus formation,
2. Adding the prepared composition that comprises the nano-size hexagonal boron nitride to the toothpaste, tooth powder, dental gel and mouthwash,
3. Applying to the teeth the nano-size hexagonal boron nitride composition added to the toothpaste, tooth powder,'dental gel and mouthwash, thereby enabling said composition to find its way into the pores on the tooth surface and also into the pores of the plaque formed on the tooth in order to prevent the crystallization and formation of the dental calculus, as well as enabling said composition to coat the tooth pores in order to prevent the tooth decay,
4. Impeding the gingival diseases and the tooth decays, owing to the application of the composition for preventing the dental calculus formation to the teeth.

According to the present invention, the nano-size hBN composition is prepared to prevent the formation of dental calculus. The composition which includes nano hBN may comprise more than 90 percent hBN and less than 10 percent boron oxide.

The D50 particle size of the nano hBN present in the composition is smaller than 500 nanometers, preferably smaller than 200 nanometers. 1 g of said composition occupies an area of 10 square meters. The nano-size hBN composition is applied at a ratio of less than 1% to the toothpaste, dental gel, tooth powder and mouthwash. The nano-size hBN added to the tooth powder is thoroughly mixed via mechanical methods, while the nano hBN composition added to the toothpaste, dental gel and mouthwash is thoroughly mixed via ultrasonic and/or mechanical methods. When the nano hBN-containing tooth powder, toothpaste, dental gel and mouthwash are applied to the teeth, the nano hBN particles that they contain find their way into the pores on the teeth surface and into the pores within the plaque formed on the teeth. The composition prevents the deposit, accumulation, formation and crystallization of the dental calculus in the regions where it settles.

Owing to the present invention, the formation of the dental calculus is prevented and the gingival diseases and the tooth decays resulting from the dental calculus are avoided by the application of the nano hBN composition, which is added to the tooth powder, dental gel, toothpaste and mouthwash, to the teeth.

## Claims

1. The present invention is a composition comprising the nano-size hBN for preventing the formation of dental calculus **characterized in that** it comprises a developed nano hBN composition.

2. Composition comprising the nano-size hBN for preventing the formation of dental calculus, according to Claim 1, **characterized in that** the composition with nano hBN ingredient comprises more than 90% nano hBN and less than 10% boron oxide.

3. Composition comprising the nano-size hBN for preventing the formation of dental calculus, according to Claims 1 and 2, **characterized in that** it comprises the nano-size hBN having a D50 particle size of smaller than 500 nanometers, preferably smaller than 200 nanometers.

4. Composition comprising the nano-size hBN for preventing the formation of dental calculus, according to Claims 1, 2 and 3, **characterized in that** it is added at a ratio of less than 1% to the tooth powder, toothpaste, dental gel and mouthwash.

5. Composition comprising the nano-size hBN for preventing the formation of dental calculus, according to Claims 1, 2, 3 and 4, **characterized in that** it is prepared by way of mixing with the tooth powder via mechanical methods, and of mixing with the toothpaste, dental gel and mouthwash via ultrasonic and/or mechanical methods.

6. Composition comprising the nano-size hBN for preventing the formation of dental calculus, according to Claims 1, 2, 3, 4 and 5, **characterized in that**
• when applied to the teeth as a tooth powder, toothpaste, dental gel and mouthwash, it finds its way into the pores on the teeth surface and into the pores within the plaque,
• it prevents the formation of the dental calculus on the tooth surface and the plaque and also the accumulation and the crystallization of the same, and
• in this way, it prevents the gingival diseases and tooth decays.
